# EUROPÄISCHE PATENTSCHRIFT

(11) **EP 1 864 642 B1**
(45) Veröffentlichungstag und Bekanntmachung des Hinweises auf die Patenterteilung: **08.10.2008**
(21) Anmeldenummer: 06115108.0
(22) Anmeldetag: 07.06.2006
(51) Int. Cl.: A61K 6/00, A61K 6/083

(54) **Dentalmaterialien auf der Basis von ringöffnend polymerisierbaren, aciden Monomeren**
Dental material based on ring-openable, polymerisable, acidic monomers
Matériau dentaire à base de monomères acides comprenant des cycles ouvrables polymérisables

(43) Veröffentlichungstag der Anmeldung: 12.12.2007
(73) Patentinhaber: Ivoclar Vivadent AG, FL-9494 Schaan (LI)
(72) Erfinder: Moszner, Norbert, Prof. Dr., 9495, Triesen (LI); Angermann, Jörg, Dr., 6800, Feldkirch (AT); Zeuner, Frank, Dr., 9488, Schellenberg (LI); Fischer, Urs-Karl, 9320, Arbon (CH); Rheinberger, Volker, Dr., 9490, Vaduz (LI); Meijere, Armin, de, Prof. Dr., 37077, Göttingen (DE)
(74) Vertreter: UEXKÜLL & STOLBERG

(56) Entgegenhaltungen:
- EP-A- 1 413 569
- EP-A1- 1 083 179
- WO-A-96/19471
- WO-A2-20/04078100
- MOSZNER N: "New monomers for dental application" MACROMOLECULAR SYMPOSIA, Bd. 217, 2004, Seiten 63-75, XP002406197
- MOSZNER, N.; SALZ, U.; ZIMMERMANN, J.: "Chemical aspects of self-etching enamel-dentin adhesives: A systematic review" DENTAL MATERIALS, Bd. 21, 2005, Seiten 895-910, XP002406198

## Beschreibung

Die Erfindung betrifft Dentalmaterialien auf der Basis von ringöffnend polymerisierbaren, aciden Monomeren. Die Dentalmaterialien eignen sich besonders zur Herstellung von Kompositen, Zementen, Adhäsiven oder Beschichtungen.

Radikalisch polymerisierbare cyclische Monomere zeichnen sich im Vergleich zu linearen Monomeren durch einen deutlich geringeren Polymerisationsschrumpf aus (vgl. R. K. Sadhir, R. M. Luck, Expanding Monomers, CRC Press, Boca Raton etc. 1992). Bei Dentalmaterialien kann der Polymerisationsschrumpf u.a. zu nachteiligen Schrumpfungsspannungen und zur Randspaltbildung bei Füllungskompositen, zur verminderten Substrathaftung bei Befestigungskompositen und Beschichtungsmaterialien sowie zur Beeinträchtigung der Dimensionsstabilität von Prothesenkunststoffen führen. Dementsprechend haben schrumpfungsarme Monomere im Dentalbereich großes Interesse gefunden (vgl. N. Moszner, U. Salz, Progress Polymer Sci. 26 (2001) 535-576).

Zur Verbesserung der Haftung von dentalen Füllungskompositen an der Zahnhartsubstanz, verwendet man diese in Kombination mit Schmelz-Dentin-Haftvermittlern. Dabei finden zunehmend sogenannte selbstätzende Schmelz-Dentin-Adhäsive Anwendung, bei denen auf eine bei der Säureätztechnik übliche Vorbehandlung der Zahnhartsubstanz mit Phosphorsäure verzichtet werden kann (N. Moszner, U. Salz, J. Zimmermann, Dental Mater. 21 (2005) 895-910). Derartige Adäsive basieren auf Haftmonomeren, die neben einer radikalisch polymerisierbaren Gruppe, meistens einer (Meth)acrylat-Gruppe, eine stark saure Haftgruppe, wie z.B. Phosphonsäure- oder Dihydrogenphosphatgruppen, enthalten. Neuerdings werden auf Basis dieser Monomere auch Komposite hergestellt, die selbsthaftende Eigenschaften zeigen und sich damit besonders als Befestigungszement eignen. Nachteilig an diesen Monomeren ist, daß sie sich nicht mit anderen Monomeren vertragen. Beispielsweise sind die bekannten radikalisch ringöffnenden cyclischen Monomere, wie Spiroorthoester, Spiroorthocarbonate oder cyclische Ketencetale in Gegenwart von starken Säuren nicht lagerstabil und können daher nicht mit diesen zusammen eingesetzt werden. Gleichfalls lassen sich auf der Basis typischer kationisch ringöffnender cyclischer Monomere, wie Glycidylethern, cycloaliphatischen Epoxiden oder Oxetanen oder anderen cyclischen Ethern, in Kombination mit den oben genannten stark sauren Monomeren keine bei Raumtemperatur lagerstabilen homogenen Mischungen herstellen.

Die WO 2004/078100 offenbart radikalisch polymerisierbare Phosphorsäureester, die unter aciden Bedingungen hydrolysestabil und zur Herstellung von selbstätzenden Dentalmaterialien geeignet sein sollen.

EP 1 413 569 offenbart polymerisationsfähige, bicyclische Cyclopropanderivate, die eine mit Methacrylaten vergleichbare radikalische Polymerisationsfähigkeit aufweisen, und deren Verwendung zur Herstellung von Dentalmaterialien.

Moszner, Macromol. Symp. 2004, 217, 63-75, beschreibt die Synthese von bicyclischen Cyclopropylacrylaten mit geringem Polymerisationsschrumpf und verbesserter Reaktivität. Außerdem wird die Herstellung von hydrolysestabilen, polymerisierbaren Acryletherphosphonsäuren und vernetzenden Bisacrylamiden beschrieben, die sich besonders zur Herstellung selbstätzender Dentalmaterialien eignen.

Der Erfindung liegt daher die Aufgabe zugrunde, Dentalmaterialien bereitzustellen, die sowohl selbsthaftende Eigenschaften als auch einen geringen Polymerisationsschrumpf in Kombination mit guten mechanischen Eigenschaften und hohler Reaktivität aufweisen.

Diese Aufgabe wird durch Dentalmaterialien gelöst, die mindestens ein radikalisch polymerisierbares Monomer gemäß der allgemeinen Formel (I) enthalten, 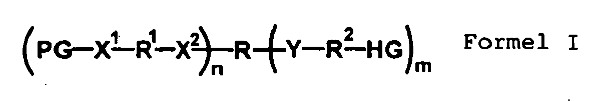 in der
- PG =: eine cyclische, ringöffnend polymerisierbare Gruppe;
- X¹ =: entfällt, O, S, eine Ester-, Amid- oder Urethangruppe;
- X² =: entfällt, O, S, eine Ester-, Amid- oder Urethangruppe;
- Y =: entfällt, O, S, eine Ester-, Amid- oder Urethangruppe;
- R =: ein (n+m)-wertiger organischer Rest mit 1 bis 35 Kohlenstoffatomen und 0 bis 8 Heteroatomen, dessen H-Atome ganz oder teilweise durch F-Atome substituiert sein können;
- R¹ =: entfällt oder ein C₁-C₁₆-Alkylenrest, der durch O-Atome unterbrochen sein kann;
- R² =: entfällt oder ein C₁-C₁₆-Alkylenrest, der durch O-Atome unterbrochen sein kann;
- n =: 1, 2, 3, 4, 5 oder 6;
- m =: 1, 2 oder 3;
- HG =: -P=O (OH) ₂; -P=O (OH) (OR¹⁴) ; -O-P=O (OH) ₂, -O-P=O (OH) (OR¹⁵) oder -SO₂OH mit
- R¹⁴ =: ein C₁-C₁₅-Alkylrest, Phenyl- oder Benzyl-Rest;
- R¹⁵ =: ein C₁-C₁₅-Alkylrest, Phenyl- oder Benzyl-Rest.

Diese Verbindungen enthalten neben einer ringöffnend polymerisierbaren Gruppe PG eine acide Gruppe HG. Durch die Formel (I) und die übrigen hierin gezeigten Formeln werden sämtliche konstitutions- und stereoisomere Formen sowie Gemische verschiedener konstitutions- und stereoisomerer Formen, wie z.B. Racemate, erfaßt. Die Formeln erfassen nur solche Verbindungen, die mit der chemischen Valenzlehre vereinbar sind.

Der Hinweis, daß eine Rest z.B. durch O unterbrochen sein kann, ist so zu verstehen, daß diese Atome oder Gruppen in die Kohlenstoffkette des Restes eingeschoben werden, d.h. beidseitig von Kohlenstoffatomen begrenzt werden. Die Anzahl dieser Fremdatome oder Gruppen ist daher um mindestens 1 kleiner als die Zahl der Kohlenstoffatome, und die Fremdatome oder Gruppen können nicht endständig sein. Erfindungsgemäß sind in allen Fällen, in denen Reste Heteroatome enthalten können, Reste ohne Heteroatome bevorzugt.

R kann ein alicyclischer, aliphatischer oder aromatischer Rest oder eine Kombination davon sein. Unter Kombinationen werden beispielsweise Alkylen-Arylen-, Alkylen-Arylen-Alkylen- und Arylen-Alkylen-Arylen-Gruppen, insbesondere -CH₂-Ph- und -CH₂-Ph-CH₂-Gruppen verstanden. Die in R gegebenenfalls vorhandenen Heteroatome sind vorzugsweise O und/oder N. Die Wasserstoffatome von R können ganz oder vorzugsweise teilweise durch F-Atome substituiert sein. Bevorzugt sind Reste ohne Fluorsubstituenten.

Die ringöffnend polymerisierbare Gruppe PG ist eine Gruppe mit einer der folgenden Formeln: 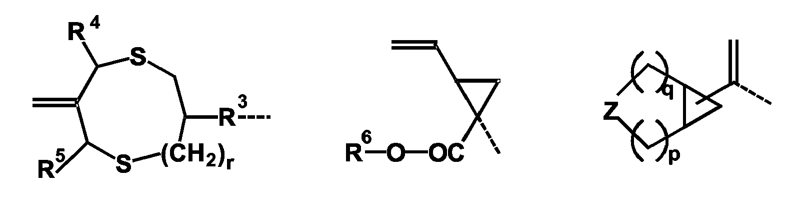 in denen
- Z =: O, S, 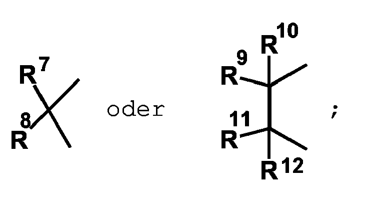
- R³ =: entfällt oder ein C₁-C₁₆-Alkylenrest, der durch O-Atome unterbrochen sein kann;
- R⁴ =: H oder ein C₁₀-Alkylrest;
- R⁵ =: H oder ein C₁-C₁₀-Alkylrest;
- R⁶ =: H, ein C₁-C₁₀-Alkylrest, Phenylrest oder Benzylrest;
- R⁷ =: H, CN, CO-OR¹³, -CO-R¹³ oder R¹³;
- R⁸ =: H, CN, CO-OR¹³, -CO-R¹³, oder R¹³;
- R⁹-R¹² =: unabhängig voneinander jeweils H, -CO-OR¹³, -CONHR¹³, -CO-R¹³, ein C₁-C₁₅-Alkylrest, der durch O unterbrochen sein kann, ein cycloaliphatischer C₄-C₁₂-Rest, ein bicyclischer C₅-C₁₂-Rest, ein C₆-C₁₄-Arylrest oder C₇-C₂₀-Alkylarylrest;
- R¹³ =: ein C₁-C₁₅-Alkylrest, der durch O unterbrochen sein kann, ein cycloaliphatischer C₄-C₁₂-Rest, ein bicyclischer C₅-C₁₂-Rest, ein C₆-C₁₄-Arylrest oder C₇-C₂₀-Alkylarylrest, wobei mehrere Reste R¹³ gleich oder verschieden sein können;
- q =: 0, 1, 2 oder 3;
- p =: 0, 1 oder 3;
- r =: 0 oder 1.

Besonders bevorzugt sind ringöffnend polymerisierbare Monomere, bei denen die Variablen der oben gezeigten polymerisierbaren Gruppen die folgende Bedeutung haben, wobei diese Bedeutungen unabhängig voneinander gewählt werden können:
- R³ =: entfällt oder ein C₁-Cₗₒ-Alkylenrest, der durch O-Atome unterbrochen sein kann, insbesondere - (CH₂) ₁₀-, -CH₂-CH₂-oder -CH₂CH₂-O-CH₂CH₂-;
- R⁴ =: H oder ein C₁-C₅-Alkylrest;
- R⁵=: H oder ein C₁-C₅-Alkylrest;
- R⁶ =: C₁-C₅-Alkylrest, ein Phenylrest oder Benzylrest; insbesondere ein C₁-C₃-Alkylrest oder Phenylrest;
- R⁷ =: H, CO-OR¹³, -CO-R¹³ oder R¹³;
- R⁸ =: H, CO-OR¹³, -CO-R¹³, oder R¹³;
- R⁹-R¹² =: unabhängig voneinander jeweils H, -CO-OR¹³, -CO-R¹³, ein C₁-C₁₅-Alkylrest, der durch O unterbrochen sein kann, ein cycloaliphatischer C₄-C₆-Rest, ein bicyclischer C₅-C₁₂-Rest, ein Phenylrest, oder Benzylrest, insbesondere H, -CO-OC₂H₅, -CO-Phenyl, C₁-C₅-Alkyl, ein bicyclischer C₅-C₁₀-Rest oder ein Benzyl-Rest;
- R¹³ =: ein C₁-C₁₀-Alkylrest, der durch O unterbrochen sein kann, ein cycloaliphatischer C₄-C₆-Rest, ein Phenyl-, oder Benzyl-Rest, insbesondere Methyl, Ethyl, Propyl, Phenyl und Benzyl, wobei mehrere Reste R¹³ gleich oder verschieden sein können;
- q =: 0, 1 oder 2;
- p =: 0, 1, 2 oder 3;
- r =: 0 oder 1.

Ganz besonders bevorzugt sind Monomere, bei denen PG eine Gruppe gemäß einer der folgenden Formeln ist:

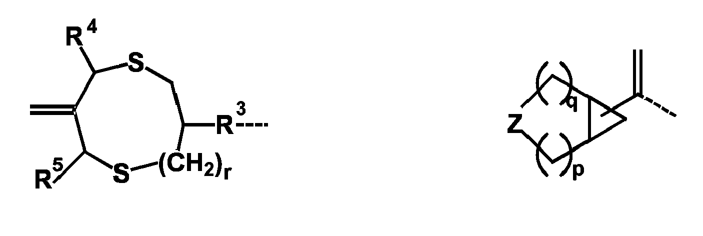

Bevorzugte Definitionen der übrigen Variablen der Formel (I), die unabhängig voneinander gewählt werden können , sind:
- X¹=: entfällt, O, eine Ester-, Amid- oder Urethangruppe, insbesondere eine Ester- Amid- oder Urethangruppe;
- X² =: entfällt, O, eine Ester-, Amid- oder Urethangruppe, insbesondere eine Ester- Amid- oder Urethangruppe;
- Y =: entfällt, O, eine Ester-, Amid- oder Urethangruppe, insbesondere eine Ester- Amid- oder Urethangruppe;
- R =: ein (n+m)-wertiger, cyclischer oder aliphatischer organischer Rest mit 1 bis 20 Kohlenstoffatomen und 0 bis 8, vorzugsweise 0 bis 4 Heteroatomen, dessen H-Atome ganz oder teilweise durch F-Atome substituiert sein können;
- R¹=: entfällt oder ein C₁-C₁₂-Alkylenrest, der durch O-Atome unterbrochen sein kann;
- R² =: entfällt oder ein C₁-C₁₂-Alkylenrest, der durch O-Atome unterbrochen sein kann;
- n =: 1, 2 oder 3;
- m =: 1, 2 oder 3;
- HG =: -P=O(OH)₂; -O-P=O (OH) oder -SO₂OH.

Besonders bevorzugt sind Monomere, bei dem mindestens eine der Variablen eine der folgenden bevorzugten Bedeutungen hat:
- X¹ =: entfällt oder eine Estergruppe;
- X² =: entfällt;
- Y =: entfällt;
- R =: ein aliphatischer, vorzugsweise linearer organischer Rest mit 2 bis 12 Kohlenstoffatomen, dessen H-Atome ganz oder teilweise durch F-Atome substituiert sein können, oder Phenylen;
- R¹ =: entfällt;
- R² =: entfällt, Methylen oder Ethylen;
- n =: 1 oder 2;
- m =: 1;
- HG =: -P=O(OH)₂; -O-P=O (OH)₂ oder -SO₂OH.

Besonders bevorzugt sind naturgemäß solche Verbindungen, bei denen alle Variablen eine der bevorzugten und insbesondere der besonders bevorzugten Bedeutungen haben.

Die Monomeren der allgemeinen Formel (I) können ausgehend von geeignet funktionalisierten cyclischen Monomeren durch Umsetzung mit entsprechenden säuregruppenhaltigen Verbindungen erhalten werden.

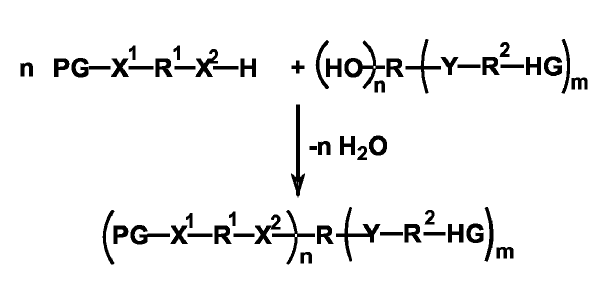

Konkretes Beispiel (n, m = 1, X¹, Y, R¹ und R² entfallen, PG = 3-Methylen, X² = C-CO, HG = O-PO(OH)₂):

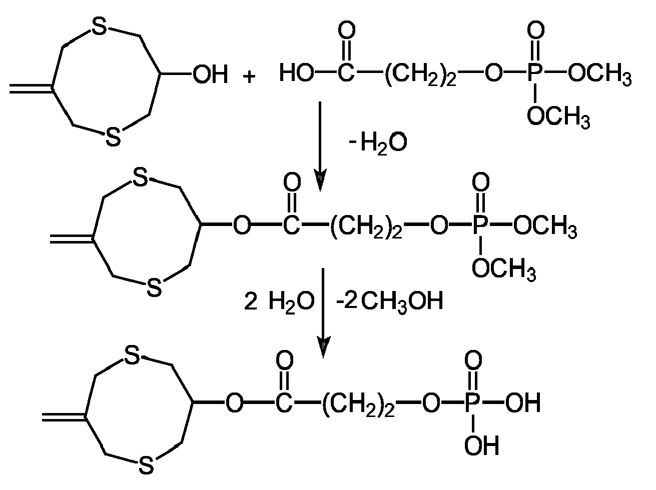

Bei der Reaktion sind Aspekte der Schutzgruppentechnik zu berücksichtigen, d.h. zuerst wird der Phosphor-, Phosphon- oder Sulfonsäurester mit den geeignet funktionalisierten cyclischen Monomeren verknüpft und anschließend die Säuregruppe freigesetzt.

Geeignet funktionalisierte cyclische Monomere für die Synthese von Monomeren der allgemeinen Formel I sind aus der Literatur bekannt. Beispielsweise wird die Synthese von Vinylcyclopropanen und von bicyclischen Cyclopropylacrylaten von N. Moszner et al., Macromol. Rapid. Commun. 18 (1997) 775-780, bzw. A. de Meijere et al., Eur.J. Org. Chem, 2004, 3669-3678, beschrieben. Die Synthese von funktionalisierten cyclischen Allylsulfiden wurde von R.A. Evans und E. Rizzardo in J. Polym. Sci., Part A. Polym. Chem. 39 (2001) 202-215; Macromolecules 33 (2000) 6722-6731, publiziert.

Bevorzugte Beispiele der ringöffnend polymerisierbaren aciden Monomeren der allgemeinen Formel I sind:

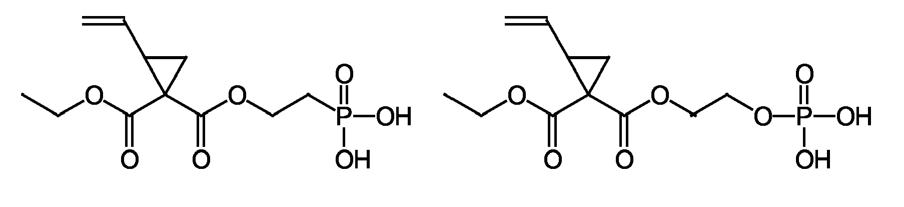

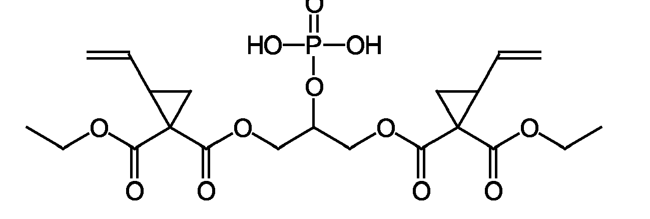

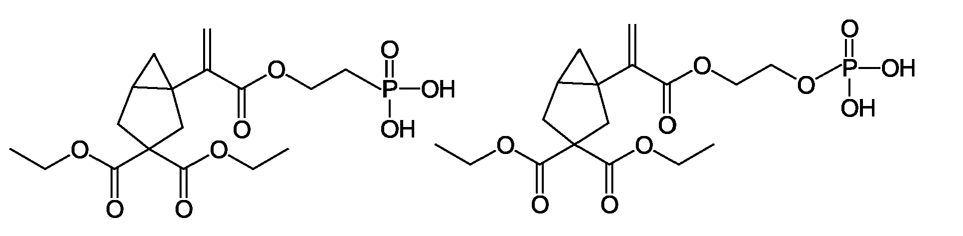

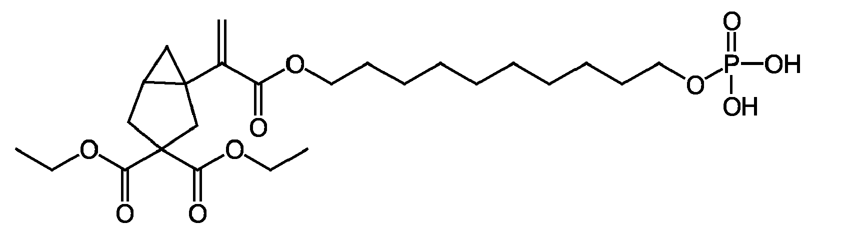

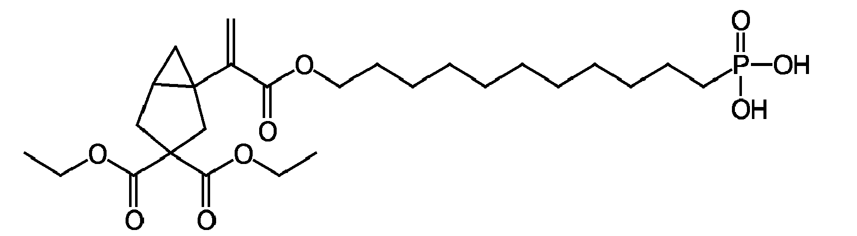

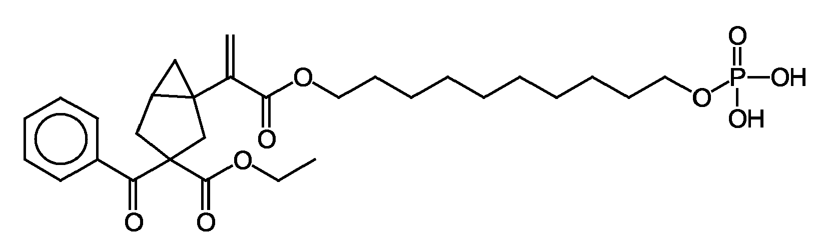

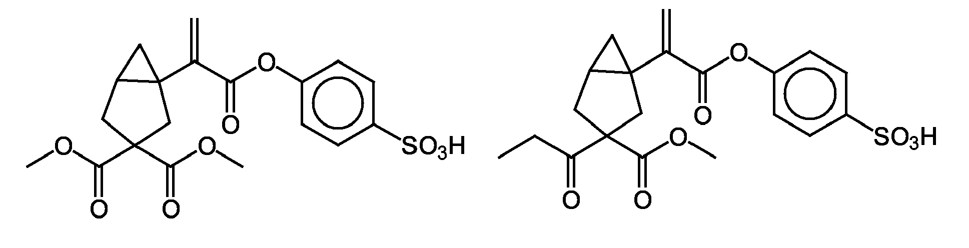

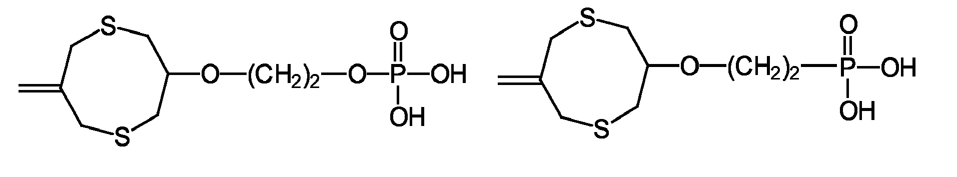

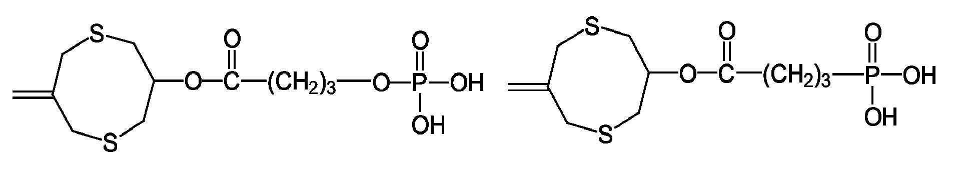

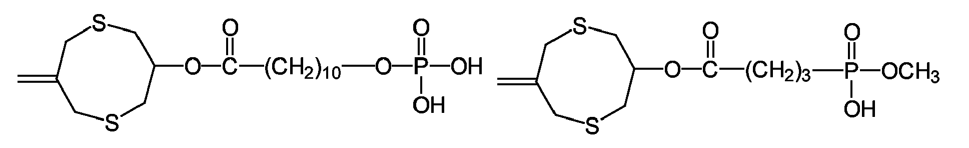

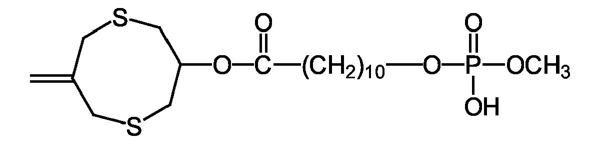

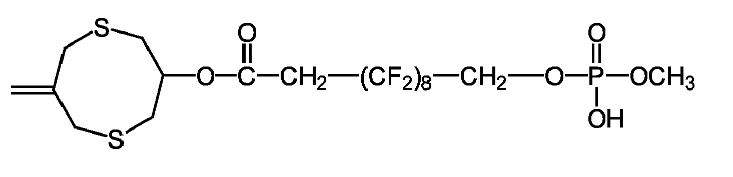

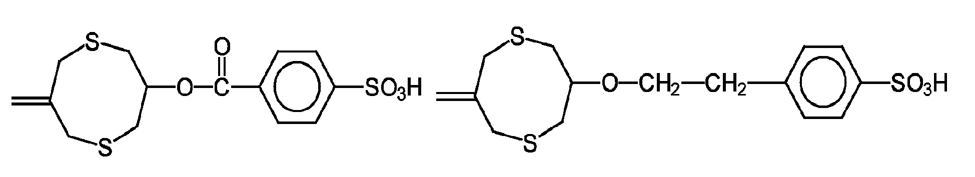

Die erfindungsgemäßen Dentalmaterialien auf Basis der Monomere der Formel (I) lassen sich mit den bekannten radikalischen Initiatoren polymerisieren, wie beispielsweise den in Encyclopedia of Polymer Science and Engineering, Vol. 13, Wiley-Intersci. Pub., New York etc. 1988, 754ff. beschriebenen. Es eignen sich besonders Photoinitiatoren, wie die aus J.P. Fouassier, J.F. Rabek (Hrsg.), Radiation Curing in Polymer Science and Technology, Vol. II, Elsevier Applied Science, London und New York 1993, bekannten. Für den UV- oder sichtbaren Bereich sind Benzoinether, Dialkylbenzilketale, Dialkoxyacetophenone, Acylder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon als Photoinitiatoren bevorzugt.

Weiterhin können auch Azoverbindungen, wie 2,2'-Azobis(iso-butyronitril) (AIBN) oder Azobis-(4-cyanovaleriansäure), oder Peroxide, wie Dibenzoylperoxid, Dilauroylperoxid, tert.-Butylperoctoat, tert.-Butylperbenzoat oder Di-(tert.-butyl)-peroxid als Initiatoren für die radikalische Polymerisation verwendet werden. Als Initiatoren für die Heißhärtung eignen sich besonders Benzpinakol und 2,2'-Dialkylbenzpinakole.

Zur Beschleunigung der Initiierung werden Peroxide und α-Diketone vorzugsweise in Kombinationen mit aromatischen Aminen eingesetzt. Bevorzugte Redoxsysteme sind Kombinationen aus Benzoylperoxid oder Campherchinon mit Aminen, wie N,N-Dimethyl-p-toluidin, N,N-Dihydroxyethyl-p-toluidin, p-Dimethylaminobenzoesäureethylester oder strukturverwandten Systemen. Darüber hinaus sind auch Redoxsysteme geeignet, die Peroxide in Kombination mit Ascorbinsäure, Barbituraten oder Sulfinsäuren als Reduktionsmittel enthalten.

Die erfindungsgemäßen Dentalwerkstoffe können ein oder mehrere Monomere der Formel (I) enthalten. Sie können darüber hinaus neben den Monomeren der Formel (I) weitere radikalisch polymerisierbare Monomere mit einer oder mehreren radikalisch polymerisierbaren Gruppen enthalten. Dentalwerkstoffe, die mindestens ein weiteres radikalisch polymerisierbares Monomer mit 2 oder mehr, vorzugsweise 2 bis 3 radikalisch polymerisierbaren Gruppen enthalten, sind besonders bevorzugt. Mehrfach funktionelle Monomere haben vernetzende Eigenschaften.

Bevorzugte zusätzliche Monomere sind mono- oder mehrfach funktionelle (Meth)acrylate oder (Meth)acrylamide ((Meth)acrylverbindungen). Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden.

Bevorzugte mehrfach funktionelle Monomere sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrit-tetra(meth)acrylat, sowie Butandioldi(meth)acrylat, 1,10-Decandioldi(meth)acrylat oder 1,12-Dodecandioldi(meth)acrylat.

Die erfindungsgemäßen Dentalwerkstoffe enthalten neben dem Monomer der Formel (I) vorzugsweise mindestens ein weiteres radikalisch polymerisierbares, säuregruppenhaltiges Monomer. Diese säuregruppenhaltige Monomere werden im Folgenden auch als acide Monomere bezeichnet. Bevorzugte Säuregruppen sind Carbonsäuregruppen, Phosphonsäuregruppen, Phosphatgruppen und/oder Sulfonsäuregruppen, wobei diese Gruppen in der Säureform oder in Form eines Esters vorliegen können. Besonders bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure und das entsprechende Anhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure und 2-[2-Dihydroxyphosphoryl)-ethoxymethyl]-acrylsäure-2,4,6-trimethyl-phenylester.

Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritolpentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Bevorzugte polymerisationsfähige Sulfonsäuren sind Vinylsulfonsäure, 4-Vinylphenylsulfonsäure oder 3-(Methacrylamido)-propylsulfonsäure.

Ganz besonders bevorzugt sind Mischungen von aciden Monomeren der Formel (I) mit bekannten schrumpfungsarmen radikalisch ringöffnend polymerisierbaren Monomeren wie mono- oder multifunktionellen Vinylcyclopropanen bzw. bicyclischen Cyclopropanderivaten, vorzugsweise den in DE 196 16 183 C2 und EP 1 413 569 offenbarten Monomeren, oder cyclischen Allylsulfiden, vorzugsweise den in US 6,043,361 und US 6,344,556 offenbarten Monomeren. Weiter bevorzugt sind außerdem Mischungen von aciden Monomeren der Formel (I) mit mindestens einem weiteren ringöffnend polymerisierbaren Monomer und mindestens einem radikalisch polymerisierbaren Monomer mit zwei oder mehr radikalisch polymerisierbaren Gruppen, insbesondere den oben aufgeführten mehrfach funktionellen (Meth)acrylatverbindungen.

Besonders bevorzugte ringöffnend polymerisierbare Monomere sind Vinylcyclopropane, wie 1,1-Di(ethoxycarbonyl- oder 1,1-Di(methoxycarbonyl)-2-vinylcyclopropan oder die Ester der 1-Ethoxycarbonyl- oder 1-Methoxycarbonyl-2-vinylcyclopropan"carbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl-oder ethylester oder deren Disubstitutionsprodukte in 3-stellung wie (3,3-Bis(ethoxycarbonyl)bicyclo[3.1.0]hex-1-yl)acrylsäuremethyl- oder ethylester. Bevorzugte cyclische Allylsulfide sind vor allem die Additionsproduktes von 2-(Hydroxymethyl)-6-methylen-1,4-dithiepan oder 7-Hydroxy-3-methylen-1,5-dithiacylooctan mit 2,2,4-Trimethylhexymethylen-1,6-disisocyanat oder dem asymmetrischen Hexamethylendiisocyanat-Trimeren Desmodur VP LS 2294 der Bayer AG.

Weiterhin können die erfindungsgemäßen Dentalmaterialien einen oder mehrere Füllstoffe enthalten, vorzugsweise organische oder anorganische partikuläre Füllstoffe. Bevorzugte anorganische partikulären Füllstoffe sind amorphe kugelförmige nanopartikuläre Füllstoffe auf der Basis von Oxiden, wie pyrogene Kieselsäure oder Fällungskieselsäure, ZrO₂ und TiO₂ oder Mischoxiden aus SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengrösse von 0,2 bis 5 µm sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Glasfasern, Polyamid- oder Kohlenstoff-Fasern eingesetzt werden.

Schließlich lassen sich den erfindungsgemäßen Dentalmaterialien auf der Basis von Monomeren der Formel (I) im Bedarfsfalle weitere Additive zusetzen, wie z.B. Stabilisatoren, UV-Absorber, Farbstoffe oder Pigmente sowie Lösungsmittel oder Gleitmittel.

Die erfindungsgemäßen Dentalmaterialien enthalten vorzugsweise:
(a) 0,5 bis 30 Gew.-% acides ringöffnend polymerisierbares Monomer gemäß Formel (I),
(b) 0,01 bis 5 Gew.-% Initiator,
(c) 5 bis 90 Gew.-% weiteres radikalisch polymerisierbares Monomer, vorzugsweise 5 bis 90 Gew.-% weiteres ringöffnend polymerisierbares Monomer und 0 bis 50 Gew.-% mehrfach funktionelles (Meth)acrylat,
(d) 0 bis 85 Gew.-% Füllstoff,
(e) ggf. 0,01 bis 5 Gew.-% Additiv und
(f) 0 bis 50 Gew.-% Lösungsmittel.

Alle Prozentangaben beziehen sich, wenn nicht anders angegeben, auf die Gesamtmasse des Dentalmaterials.

Die erfindungsgemäßen Dentalmaterialien sind Dentalwerkstoffe und eignen sich besonders als Komposit, Zement, Adhäsiv oder Beschichtungsmaterial.

Dentalmaterialien zur Verwendung als Adhäsiv enthalten bevorzugt:
(a) 1 bis 30 Gew.-% acides ringöffnend polymerisierbares Monomer gemäß Formel (I),
(b) 0,1 bis 2 Gew.-% Initiator,
(c) 5 bis 80 Gew.-% weiteres radikalisch polymerisierbares Monomer, vorzugsweise 5 bis 80 Gew.-% weiteres ringöffnend polymerisierbares Monomer und 0 bis 30 Gew.-% mehrfach funktionelles (Meth)acrylat,
(d) 0 bis 20 Gew.-% Füllstoff,
(e) ggf. 0,01 bis 50 Gew.-% Additiv und
(f) 0 bis 50 Gew.-% Lösungsmittel.

Dentalmaterialien zur Verwendung als Zemente enthalten bevorzugt:
(a) 1 bis 30 Gew.-% acides ringöffnend polymerisierbares Monomer gemäß Formel (I),
(b) 0,1 bis 2 Gew.-% Initiator,
(c) 5 bis 30 Gew.-% weiteres radikalisch polymerisierbares Monomer, vorzugsweise 5 bis 30 Gew.-% weiteres ringöffnend polymerisierbares Monomer und 0 bis 10 Gew.-% mehrfach funktionelles (Meth)acrylat,
(d) 0 bis 70 Gew.-% Füllstoff und
(e) ggf. 0,01 bis 5 Gew.-% Additiv.

Dentalmaterialien zur Verwendung als Komposit enthalten bevorzugt:
(a) 1 bis 20 Gew.-% acides ringöffnend polymerisierbares Monomer gemäß Formel (I),
(b) 0,1 bis 2 Gew.-% Initiator,
(c) 5 bis 20 Gew.-% weiteres radikalisch polymerisierbares Monomer, vorzugsweise 5 bis 20 Gew.-% weiteres ringöffnend polymerisierbares Monomer und 0 bis 10 Gew.-% mehrfach funktionelles (Meth)acrylat,
(d) 0 bis 85 Gew.-% Füllstoff und
(e) ggf. 0,01 bis 2 Gew.-% Additiv.

Die vorliegende Erfindung betrifft auch die Verwendung von radikalisch polymerisierbaren Monomeren der Formel (I) zur Herstellung von Dentalwerkstoffen, vorzugsweise den oben beschriebenen Dentalwerkstoffen.

Außerdem betrifft die Erfindung ein Verfahren zur Herstellung von Formkörpern, wie Kronen, Brücken, Inlays und künstlichen Zähnen, bei dem man einen erfindungsgemäßen Dentalwerkstoff auf an sich bekannte Weise zu dem Formkörper formt und dann zumindest teilweise, vorzugsweise vollständig, härtet. Die Härtung erfolgt vorzugsweise durch radikalische Polymerisation.

Die Erfindung wird im Folgenden anhand von Beispielen näher erläutert.

### Ausführungsbeispiele

### Beispiel 1: Synthese eines ringöffnend polymersierbaren Dihydrogenphosphates

### 1. Stufe: Kohlensäure-O-[5,5-bis(ethoxycarbonyl)oct-7-en-2-inyl]-O'-methylester (1)

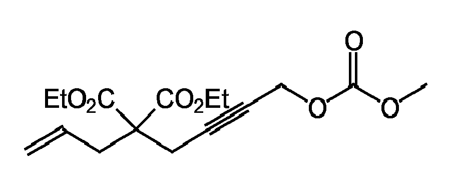

Zu einer gerührten Suspension von Natriumhydrid (1,9 g 60%-ige Dispersion in Mineralöl, 47,5 mmol) in wasserfreiem DMF (45 mL) gab man Allylmalonsäurediethylester (9,01 g, 45 mmol) hinzu. Dann kühlte man das Reaktionsgemisch auf 10 °C ab und tropfte innerhalb von 30 Min. Kohlensäure-0-(4-chlorbut-2-inyl)-O -methylester (7,4 g, 46 mmol), der nach A. Steinig, A. de Meijere, (Eur. J. Org. Chem. 1999, 1333-1344) hergestellt worden war, hinzu. Man ließ 24 Std. bei Raumtemperatur rühren und destillierte das Lösungsmittel i. Vak. ab (T_{Bad} < 60 °C, 0,1 mbar). Der Rückstand wurde in 200 ml Ether gelöst, mit 50 ml 5%-iger Schwefelsäure, 50 mL Wasser und 50 mL gesättigter NaCl-Lösung gewaschen und dann über Magnesiumsulfat getrocknet. Das Lösungsmittel wurde im Vak. wieder abdestilliert und das Rohprodukt (14,3 g) chromatographisch (100 ml Flash-Kieselgel mit Pentan/Ether 10:1 bis 5:1) gereinigt. Ausbeute: 8.1 g (55%), farbloses bis schwach-gelbes Öl.

¹H-NMR (250 MHz, CDCl₃) : δ = 1,23 (t, 6H, 2 CH₃), 2,76 (ddd, 2H, 6-H), 2,82 (t, 2H, 4-H), 3,79 (s, 3H, CH₃), 4,19 (q, 4H, 2 CH₂), 4,67 (t, 2H, 1-H), 5,10 (ddd, 1H, 8-H*ₜᵣₐₙₛ*), 5,15 (ddd, 1H, 8-H*_{cis}*), 5,60 (ddd, 1H, 7-H).

### 2. Stufe: 2-[3,3-Bis(ethoxycarbonyl)bicyclo[3.1.0]hex-1-yl)-acrylsäure (2)

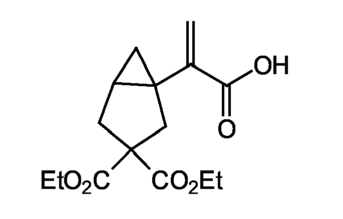

Zu einer Lösung von Tris(2-furyl)phosphin (766 mg, 3,30 mmol), Tetramethylammoniumbromid (254 mg, 1,65 mmol), BHT (397 mg, 1,80 mmol) und 1 (= 1. Stufe, 16,3 g, 50 mmol) in entgaster 60%-iger wässriger Essigsäure (1000 ml) gab man Pd(OAc)₂ (337 mg, 1,50 mmol) unter Argon hinzu und rührte das Gemisch bei 25 °C 1 h lang. Dann ersetzte man die Reaktionsatmosphäre vollständig durch Kohlenmonoxid und rührte kräftig bei Raumtemperatur bis der Kohlensäureester **1** vollständig umgesetzt war (~24 h, DC-Kontrolle). Nach Abdestillieren des Lösungsmittels im Vakuum (40 °C/0,5 mbar) wurde der Rückstand in 200 ml Diethylether suspendiert und über Celite abfiltriert. Dann wurde das Produkt mit 0,5 M wässriger Sodalösung extrahiert (250 ml) und der wässrige Extrakt 2 mal mit je 50 ml Diethylether gewaschen, und nach Zugabe von 10 mg BHT wurde die wässrige Phase mit 12 N HC1 (ca. 4-5 ml) auf einen pH-Wert von ca. 1,0 eingestellt. Man trennte die organische Phase ab und sättigte die wässrige Schicht mit NaCl und extrahierte 3 mal mit je 50 ml Diethylether. Die vereinigten organischen Phasen wurden dann mit 100 ml gesättigter NaCl-Lösung gewaschen und über wasserfreiem Natriumsulfat getrocknet. Nach Abdestillieren des Lösungsmittels wurden 10,8 g (73% Ausbeute) eines leicht gelben viskosen Öls erhalten, das man ohne Reinigung für die nächste Stufe einsetzen konnte.

¹H-NMR (300 MHz, CDCl₃) : δ = 0,54 (dd, 1H, 6-H*_{endo}*), 0,75 (ddd, 1H, 6-H*ₑₓₒ*), 1,21 (t, 3H, CH₃) , 1,25 (t, 3H, CH₃) , 1,60 (dddd, 1H), 2,57 (dd, 1H), 2,58 (dd, 1H), 2,62 (ddd, 1H), 2,74 (d, 1H), 4,14 (q, 2H, OCH₂) , 4,19 (q, 2H, OCH₂), 5,73 (d, 1H, 3'-H*ₜᵣₐₙₛ*), 6,32 (d, 1H, 3'-H*_{cis}*) und 11,9 (br., 1H, COOH). ¹³C-NMR (75,5 MHz, CDCl₃, DEPT): δ = 14,0 (2 CH₃), 16,5 (CH₂), 24,7 (CH), 30, 8 (C), 35,9 (CH₂) , 40, 1 (CH₂), 59, 9 (C), 61,6 (CH₂), 61,7 (CH₂), 128,2 (CH₂), 141,7 (C), 171,5 (C), 171,8 (C) und, 172,7 (C).

C₁₅H₂₀O₆ (296,32): ber. C 60,80, H 6,80; gef. C 60,94, H 6,80.

### 3. Stufe: 10-{2-[3,3-Bis(ethoxycarbonyl)bicyclo[3.1.0]hex-1-yl}acryloyloxy}decyldihydrogenphosphat (3)

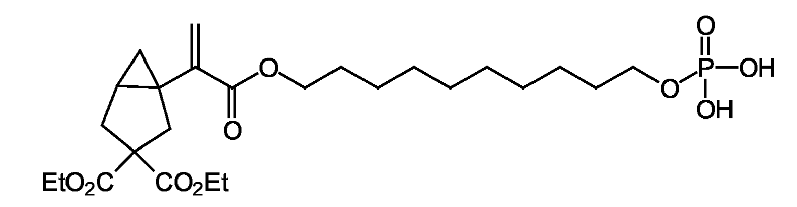

Zu einer gerührten Lösung von **2** (4,67 g, 15,8 mmol), 10-Hydroxydecyl-di-tert.-butylphosphat (5,80 g, 15,8 mmol) und Triphenylphosphin (4,25g, 16,2 mmol) in 40 mmol wasserfreiem THF wurde unter Argon und Rühren bei ca. -78 °C (Trockeneis/Aceton-Bad) Diethylazodicarboxylat (2,82 g, 16,2 mmol) so zugegeben, daß die Temperatur -70 °C nicht überstieg. Nach 30 Min. Rühren wurde das Kühlbad entfernt, die Reaktionsmischung langsam auf Raumtemperatur erwärmt und das Lösungsmittel im Vakuum abdestilliert. Anschließend wurde der Rückstand unter Rühren in 10 ml tert.-Butylmethylether suspendiert und durch Zugabe von 50 ml Pentan verdünnt. Nach Rühren im Eisbad für 1 h wurde die Suspension über eine Silicagel-Säule mit einer Mischung aus Hexan/Ethylacetat (6:1 bis 3:1) chromatographisch gereinigt. Es ergaben sich 7,95 g (78% Ausbeute) einer viskosen farblosen Flüssigkeit von 10-{2-[3,3-Bis(ethoxycarbonyl)bicyclo[3.1.0]hex-1-yl}acryloyloxy}decyl-di-tert.-bytyl-phosphat, das zur Freisetzung des entsprechenden Dihydrogenphosphates **3** in einer Mischung aus 20 ml CCl₄ und 2 ml Trifluoressigsäure gelöst wurde. Die Mischung wurde unter reduzierten Druck bei 50 °C ca. 1 h umgesetzt, wobei die Reaktionsverfolgung mittels HPLC erfolgte und sich das Produkt als hochviskose Flüssigkeit ergab.

¹H-NMR (400 MHz, DMSO-d₆) : δ = 0,52 (dd, 1H, 6-H*_{endo}*), 0,74 (ddd, 1H, 6-H*ₑₓₒ*), 1,21 (t, 3H, CH₃), 1,24 (t, 2 x 3H, CH₃), 1,26-1,40 (m, 12H, C₃-C₈-decyl) 1,51-1,68 (m, 5H, 1-H + C_{2,9}-decyl), 2,51-2,62 and 2,71 (m, 3H + 1H, both 2,4-H), 3,75-3,85 (m, 2H, CH₂OP) , 4,15 (q,2H, OCH₂), 4,17 (q,2H, OCH₂), 5,67 (d, 1H, C=H*ₜᵣₐₙₛ*), 6,09 (d, 1H, C=H*_{cis}*).

³¹P-NMR (167,5 MHz, DMSO-d₆): 2,74 (s)

### Beispiel 2: Radikalische Copolymerisation von Monomer 3 mit UDMA

Zur Bestimmung des Polymerisationsschrumpfes wurde eine Mischung aus 50 Gew.-% Monomer **3** und 50 Gew.-% UDMA (Ivoclar Vivadent AG), dem Urethandimethacrylat aus 2 Mol 2-Hydroxyethylmethacrylat und 1 Mol 2,2,4-Trimethylhexamethylendiisocyanat mit 0,3 Gew. (bezogen auf die Gesamtmischung) Kampherchinon (Photoinitiator) und 0,5 Gew.-% 4-(Dimethylamino)-benzoesäureethylester (Aminbeschleuniger) versetzt und dann mit einer dentalen Lichtquelle (Spectramat®, Ivoclar Vivadent) bestrahlt. Aus der Differenz der bestimmten Dichten der Monomermischung bzw. des gebildeten Polymerisates wurde unter Berücksichtigung des Polymerisationsschrumpfes von reinem UDMA (ΔVp = 6,1 %) ein Polymerisationsschrumpf von nur 4,4 % berechnet. Aus den Ergebnissen der Polymerisation einer analogen Mischung des Haftmonomeren 2-[4-(Dihydroxyphosphoryl)-2-oxabutyl]-acrylsäureethylester (EAEPA) und UDMA (50:50) wurde der Polymerisationsschrumpf von EAEPA zu 7,7 % berechnet.

Das Beispiel 2 zeigt, daß sich die Vinylcyclopropane bzw. Cyclopropylacrylate der Formel (I) im Vergleich zu (Meth)-acrylaten durch einen deutlich geringeren Polymerisationsschrumpf auszeichnen.

### Beispiel 3: Herstellung eines lichthärtenden Adhäsives auf der Basis von Monomer 3

Zur Untersuchung der Dentinhaftung auf Rinderzahndentin wurden Adhäsive mit der in Tabelle 1 angegebenen Zusammensetzung hergestellt:

Rinderzähne wurden so in Kunststoffzylinder eingebettet, daß sich das Dentin und der Kunststoff in einer Ebene befanden. Nach 15 s Ätzung mit 37 %-iger Phosphorsäure wurde gründlich mit Wasser abgespült. Dann wurde mit einem Microbrush eine Schicht Adhäsiv obiger Zusammensetzung aufgepinselt, mit einem Luftgebläse zur Entfernung des Lösungsmittels kurz verblasen und für 40 s mit einer Halogenlampe (Astralis 7, Ivoclar Vivadent) belichtet. Auf die Adhäsivschicht polymerisierte man einen Kompositzylinder aus Tetric^{®} Ceram (Ivoclar Vivadent) in zwei Schichten von je 1-2 mm auf. Anschließend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure zu 26,8 MPa (Adhäsiv A) bzw. 29,9 MPa (Adhäsiv B) bestimmt.

**Tabelle 1: Zusammensetzung der Adhäsive (Angaben in Gew.-%)**

| **Komponente** | **Adhäsiv A** | **Adhäsiv B (Vergleich)** |
|---|---|---|
| Monomer **3** | 10,9 | - |
| EAEPA ¹⁾ | - | 10, 9 |
| Glycerindimethacrylat | 9,9 | 9,9 |
| UDMA²⁾ | 9,9 | 9,9 |
| Bis-GMA³⁾ | 32,7 | 32,7 |
| 2-Hydroxyethylmethacrylat | 14,9 | 14,9 |
| Photoinitiator⁴⁾ | 1,7 | 1,7 |
| Ethanol (abs.) | 20,0 | 20,0 |

| | | |
|---|---|---|
| ¹⁾ 2-[4-(Dihydroxyphosphoryl)-2-oxabutyl]acrylsäureethylester ²⁾ Additionsprodukt aus 2-Hydroxyethylmethacrylat und 2,2,4-Trimethylhexamethylendiisocyanat ³⁾ Additionsprodukt aus Methacrylsäure und Bisphenol-A-diglycidylether ⁴⁾ Mischung aus Kampherchinon (0,3%), 4-Dimethyl-benzoesäureethylester (0,4%) und dem Acylphosphinoxid Lucerin TPO (1,0%) | | |

### Beispiel 4: Herstellung eines Dentalzements auf Basis des Monomers 3 aus Beispiel 1

Entsprechend der nachfolgend aufgeführten Tabelle 2 wurde ein Kompositbefestigungszement auf der Basis einer Methacrylatmischung (Probe A, Vergleich) und unter Einbeziehung der ringöffnend polymerisierbaren Phosphonsäure 3 aus Beispiel 1 (Probe B) mittels eines Walzenstuhles "Exakt (Exakt Apparatebau, Norderstedt) hergestellt. Von den Materialien wurden entsprechende Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und der Exothermzeit.

**Tabelle 2: Zusammensetzung des Kompositzements (Angaben in Gew.-%)**

| **Komponente** | **Material A (Gew.-%)** | **Material B (Gew.-%)** |
|---|---|---|
| Urethandimethacrylat¹⁾ | 31,8 | 31,8 |
| Decandioldimethacrylat | 7,8 | - |
| Monomer **3** | - | 7,8 |
| Aerosil OX-50 (Degussa) | 41,2 | 41,2 |
| Ytterbiumtrifluorid (Rhone-Poulenc) | 18,7 | 18,7 |
| Photoinitiator²⁾ | 0,5 | 0,5 |

| | | |
|---|---|---|
| ¹⁾ Urethandimethacrylat aus 2 mol 2-Hydroxyethylmethacrylat und 1 mol 2,2,4-Trimethylhexamethylendiisocyanat-1,6 ²⁾ Mischung aus Campherchinon (0,24 %), p-N,N-Dimethylaminobenzoesäureethylester (0,26 %) | | |

Aus Tabelle 3 ist ersichtlich, daß das Material B im Vergleich zum Material A (auf der Basis einer rein konventionellen Methacrylatmischung) zu fast vergleichbaren mechanischen Eigenschaften führt. Die geringe Abnahme der mechanischen Eigenschaften nach Wasserlagerung ist auf die Zunahme der Hydrophilie des Komposites durch die wasserlösliche Phosphonsäure zurückzuführen und ist für eine Verwendung im Dentalbereich nicht signifikant.

**Tabelle 3: Zementeigenschaften**

| **Materialeigenschaft** | **Material A** | **Material B** |
|---|---|---|
| Biegefestigkeit (MPa) nach 24 h | 95 | 86 |
| Biegefestigkeit (MPa) nach 24 h WL¹⁾ | 101 | 90 |
| Biegefestigkeit (MPa) nach 7 d WL | 111 | 76 |
| Biege-E-Modul (GPa) nach 24 h | 4,76 | 4,89 |
| Biege-E-Modul (GPa) nach 24 h WL | 4,93 | 4,08 |
| Biege-E-Modul (GPa) nach 7 d WL | 5,13 | 4,03 |
| Exothermzeit (s) | 13 | 8 |

| | | |
|---|---|---|
| ¹⁾ WL = Wasserlagerung der Prüfkörper | | |

## Patentansprüche

1. Dentalmaterial, das mindestens ein radikalisch polymerisierbares Monomer gemäß der allgemeinen Formel (I) enthält, 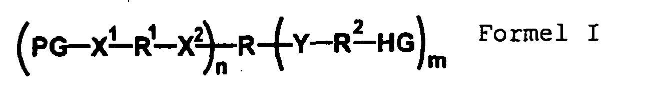 in der
PG = eine cyclische, ringöffnend polymerisierbare Gruppe mit einer der folgenden Formeln ist: 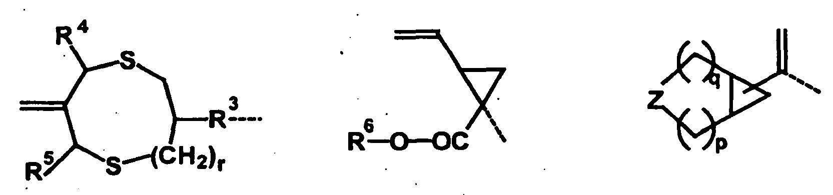 in denen
Z = O, S, 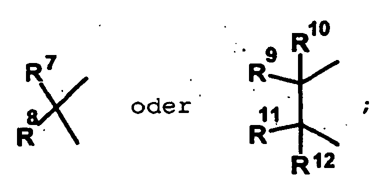
R³ = entfällt oder ein C₁-C₁₆-Alkylenrest, der durch O-Atome unterbrochen sein kann;
R⁴ = H oder ein C₁₀-Alkylrest;
R⁵ = H oder ein C₁-C₁₀-Alkylrest;
R⁶ = H, ein C₁-C₁₀-Alkylrest_{;} Phenyl- oder Benzyl-Rest;
R⁷ = H, CN, CO-OR¹³, -CO-R¹³ oder R¹³ ;
R⁸ = H, CN, CO-OR¹³, -CO-R¹³, oder R¹³
R⁹-R¹² = unabhängig voneinander jeweils H, -CO-OR¹³, -CONHR¹³, -CO-R¹³, ein C₁-C₁₅-Alkylrest, der durch O unterbrochen sein kann, ein cycloaliphatischer C₄-C₁₂-Rest, ein bicyclischer C₅-C₁₂-Rest, ein C₆-C₁₄-Arylrest oder C₇-C₂₀-Alkylarylrest;
R¹³ = ein C₁-C₁₅-Alkylrest, der durch Ounterbrochen seinkann, ein cycloaliphatischer C₄-C₁₂-Rest, ein bicyclischer C₅-C₁₂-Rest, ein C₆-C₁₄-Arylrest oder C₇-C₂₀-Alkylarylrest, wobei mehrere Reste R¹³ gleich oder verschieden sein können;
q = 0, 1, 2 oder 3;
p = 0, 1, 2 oder 3;
r = 0 oder. 1;
X¹ - entfällt, O, S, eine Ester-, Amid- oder Urethangruppe;
X² = entfällt, O, S, eine Ester-, Amid- oder Urethangruppe;
Y = entfällt, O, S, eine Ester-, Amid- oder Urethangruppe;
R = ein (n+m)-wertiger organischer Rest mit 1 bis 35 Kohlenstoffatomen und 0 bis 8 Heteroatomen, dessen H-Atome ganz oder teilweise durch F-Atome substituiert sein können;
R¹ =entfällt oder ein C₁-C₁₆-Alkylenrest, der durch O-Atome unterbrochen sein kann;
R² = entfällt oder ein C₁-C₁₆-Alkylenrest, der durch O-Atome unterbrochen sein kann;
n = 1, 2, 3, 4, 5 oder 6;
m = 1, 2 oder 3;
HG = -P=O (OH)₂ ; -P=O(OH) (OR¹⁴); -O-P=O (OH)₂, -O-P=O (OH) (OR¹⁵) oder -SO₂OH mit
R¹⁴ = ein C₁-C₁₅-Alkylrest, Phenyl- oder Benzyl-Rest;
R¹⁵ = ein C₁-C₁₅-Alkylrest, Phenyl- oder Benzyl-Rest.

2. Dentalmaterial nach Anspruch 1, bei dem mindestens eine der Variablen eine der folgenden bevorzugten Bedeutungen hat:
R³ = entfällt oder ein C₁-C₁₀-Alkylenrest, der durch O-Atome unterbrochen sein kann;
R⁴ = H oder ein C₁-C₅-Alkylrest;
R⁵ = H oder ein C₁-C₅-Alkylrest;
R⁶ = ein C₁-C₅-Alkylrest, Benzyl- oder Phenyl-Rest;
R⁷ = H, CO-OR¹³, -CO-R¹³ oder R¹³;
R⁸ = H, CO-OR¹³, -CO-R¹³, oder R¹³;
R⁹-R¹² = unabhängig voneinander jeweils H, -CO-OR¹³, -CO-R¹³, ein C₁-C₁₅-Alkylrest, der durch O unterbrochen sein kann, ein cycloaliphatischer C₄-C₆-Rest, ein bicyclischer C₅-C₁₂-Rest, ein Phenyl-, oder Benzyl-Rest;
R¹³ = ein C₁-C₁₀-Alkylrest, der durch O unterbrochen sein kan, ein cycloaliphatischer C₄-C₆-Rest, ein Phenyl-, oder Benzyl-Rest, wobei mehrere Reste R¹³ gleich oder verschieden sein können;
q = 0, 1 oder 2;
p = 0, 1 oder 3;
r = 0 oder 1.

3. Dentalmaterial nach Anspruch 1 oder 2, bei dem PG eine Gruppe der Formel 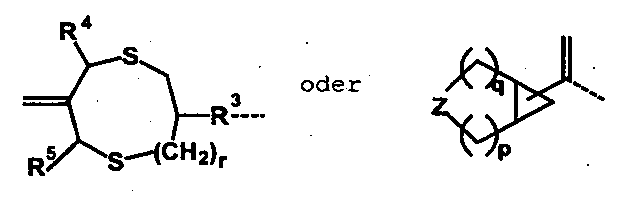 ist.

4. Dentalmaterial nach einem der vorhergehenden Ansprüche, bei dem mindestens eine der Variablen eine der folgenden bevorzugten Bedeutungen hat:
X¹ = entfällt, O, eine Ester-, Amid- oder Urethangruppe;
X² = entfällt, O, eine Ester-, Amid- oder Urethangruppe;
Y = entfällt, O, eine Ester-, Amid- oder Urethangruppe;
R = ein (n+m)-wertiger organischer Rest mit 1 bis 20 Kohlenstoffatomen und 0 bis 8 Heteroatomen, dessen H-Atome ganz oder teilweise durch F-Atome substituiert sein können;
R¹ = entfällt oder ein C₁-C₁₂-Alkylenrest, der durch O-Atome unterbrochen sein kann;
R² =entfällt oder ein C₁-C₁₂-Alkylenrest, der durch O-Atome unterbrochen sein kann;
n = 1, 2 oder 3;
m = 1, 2 oder 3;
HG = -P=O (OH)₂; -O-P=O (OH)₂ oder -SO₂OH.

5. Dentalmaterial nach Anspruch 4, bei dem mindestens eine der Variablen eine der folgenden bevorzugten Bedeutungen hat:
X¹ = entfällt oder eine Estergruppe;
X² = entfällt;
Y = entfällt;
R = ein aliphatischer organischer Rest mit 2 bis 12 Kohlenstoffatomen, dessen H-Atome ganz oder teilweise durch F-Atome substituiert sein können, oder Phenylen;
R¹ = entfällt;
R² = entfällt, Methylen oder Ethylen;
n = 1 oder 2;
m = 1;
HG = -P=O(OH)₂; -O-P=O (OH)₂ oder -SO₂OH.

6. Dentalmaterial nach einem der vorhergehenden Ansprüche, das zusätzlich einen Initiator für die radikalische Polymerisation enthält.

7. Dentalmaterial nach einem der vorhergehenden Ansprüche, das zusätzlich mindestens ein weiteres radikalisch polymerisierbares Monomer enthält.

8. Dentalmaterial nach Anspruch 7, das mindestens ein radikalisch polymerisierbares Monomer mit zwei oder mehr radikalisch polymerisierbaren Gruppen enthält.

9. Dentalmaterial nach Anspruch 8, das mindestens ein Monomer enthält, das aus bi- oder mehrfunktionellen Acrylaten und Methacrylaten, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Butandioldi(meth)acrylat, 1,10-Decandioldi(meth)acrylat, 1;12-Dodecandioldi(meth)acrylat ausgewählt ist.

10. Dentalmaterial nach einem der Ansprüche 7 bis 8, das mindestens ein radikalisch ringöffnend polymerisierbares Monomer enthält.

11. Dentalmaterial nach Anspruch 10, das mindestens ein Monomer enthält, das aus mono- und multifunktionellen Vinylcyclopropanen, bicyclischen Cyclopropanderivaten oder cyclischen Allylsulfiden ausgewählt ist.

12. Dentalmaterial nach einem der Ansprüche 7 bis 11, das mindestens ein weiteres radikalisch polymerisierbares, säuregruppenhaltiges Monomer enthält.

13. Dentalmaterial nach einem der vorhergehenden Ansprüche, das zusätzlich mindestens einen Füllstoff enthält.

14. Dentalmaterial nach einem der vorhergehenden Ansprüche, das weiterhin mindestens ein Additiv enthält, das aus Stabilisatoren, UV-Absorbern, Farbstoffen, Pigmenten, Lösungsmitteln und Gleitmitteln ausgewählt ist.

15. Dentalmaterial nach einem der vorhergehende Ansprüche, das
(a) 0,5 bis 30 Gew.-% acides ringöffnend polymerisierbares Monomer gemäß Formel (I),
(b) 0,01 bis 5 Gew.-% Initiator,
(c) 5 bis 90 Gew.-% weiteres radikalisch polymerisierbares Monomer,
(d) 0 bis 85 Gew.-% Füllstoff,
(e) ggf. 0,01 bis 5 Gew.-% Additiv und
(f) 0 bis 50 Gew.-% Lösungsmittel enthält.

16. Dentalmaterial nach Anspruch 15, das als weiteres Monomer (c) 5-90 Gew.-% zusätzliches ringöffnend polymerisierbares Monomer und 0-50 Gew.-% mehrfach funktionelles (Meth)acrylat enthält.

17. Verwendung eines radikalisch polymerisierbares Monomer gemäß der allgemeinen Formel (I) zur Herstellung eines Dentalwerkstoffs.

18. Verwendung nach Anspruch 17, wobei der Dentalwerkstoff ein Komposit, Zement, Adhäsivs oder Beschichtungsmaterial ist.

19. Verfahren zur Herstellung eines Formkörpers, bei dem man ein Dentalmaterial gemäß einem der Ansprüche 1 bis 16 zu einem Körper mit der gewünschten Form formt und anschließend ganz oder teilweise härtet.

## Claims

1. A dental material comprising at least one radically polymerisable monomer according to the general Formula (1), 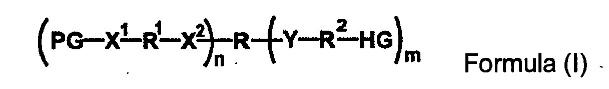 in which
PG = a cyclic, ring-opening polymerisable group with one of the following formulae: 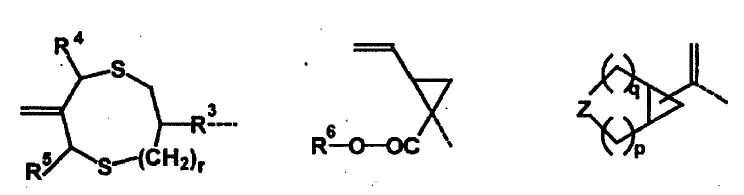 in which
Z = O, S, 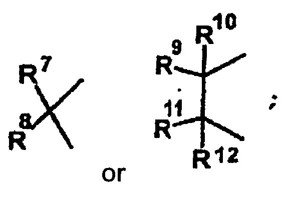
R³ = is absent or is a C₁-C₁₆ alkylene group that can be interrupted by O atoms;
R⁴ = H or a C₁₀ alkyl group;
R⁵ = H or a C₁-C₁₀ alkyl group;
R⁶ = H, a C₁-C₁₀ alkyl group, phenyl or benzyl group;
R⁷ = H, CN, CO-OR¹³ -CO-R¹³ or R¹³;
R⁸ = H, CN, CO-OR¹³ -CO-R¹³ or R¹³;
R⁹-R¹² = each independently of one another H, -CO-OR¹³, -CONHR¹³, - CO-R¹³, a C₁-C₁₅ alkyl group that can be interrupted by O, a cycloaliphatic C₄-C₁₂ group, a bicyclic C₅-C₁₂ group, a C₆-C₁₄ aryl group or C₇-C₂₀ alkylaryl group;
R¹³ = a C₁-C₁₅ alkyl group that can be interrupted by O, a cycloaliphatic C₄-C₁₂ group, a bicyclic C₅-C₁₂ group, a C₆-C₁₄ aryl group or C₇-C₂₀ alkylaryl group, wherein a plurality of **R¹³** groups can be the same or different;
q = 0, 1, 2 or 3;
p = 0, 1, 2 or 3;
r = or 1;
X¹ = is absent, O, S, an ester, amide or urethane group;
X² = is absent, O, S, an ester, amide or urethane group;
Y = is absent, O, S, an ester, amide or urethane group;
R = an (n+m)-valent organic group containing 1 to 35 carbon atoms and 0 to 8 hetero atoms, the H atoms of which optionally being totally or partially substituted by F atoms;
R¹ = is absent or a C₁-C₁₆ alkylene group that can be interrupted by O atoms;
R² = is absent or a C₁-C₁₆ alkylene group that can be interrupted by O atoms;
n = 1, 2, 3, 4, 5 or 6;
m=1,2or3;
HG = -P=O(OH)₂; -P=O(OH)(OR¹⁴); -O-P=O(OH)₂, -O-P=O(OH)(OR¹⁵) or -SO₂OH with
R¹⁴ = a C₁-C₁₅ alkyl group, phenyl or benzyl group;
R¹⁵ = a C₁-C₁₅ alkyl group, phenyl or benzyl group.

2. Dental material according to Claim 1, in which at least one of the variables has one of the following meanings:
R³ = is absent or is a C₁-C₁₀ alkylene group that can be interrupted by O atoms;
R⁴ = H or a C₁-C₅ alkyl group;
R⁵ = H or a C₁-C₅ alkyl group;
R⁶ = a C₁-C₅ alkyl group, phenyl or benzyl group;
R⁷ = H, CO-OR¹³, -CO-R¹³ or R¹³;
R⁸ = H, CO-OR¹³, -CO-R¹³ or R¹³_{;}
R⁹-R¹² = each independently of one another H, CO-OR¹³, -CO-R¹³, a C₁-C₁₅ alkyl group that can be interrupted by O, a cycloaliphatic C₄-C₆ group, a bicyclic C₅-Cₗ₂ group, a phenyl or benzyl group;
R¹³ = a C₁-C₁₀ alkyl group that can be interrupted by O, a cycloaliphatic C₄-C₆ group, a phenyl or benzyl group, wherein a plurality of R¹³ groups can be the same or different;
q = 0, 1 or 2;
p = 0, 1 or 3;
r = 0 or 1.

3. Dental material according to Claim 1 or 2, in which PG is a group of the Formula

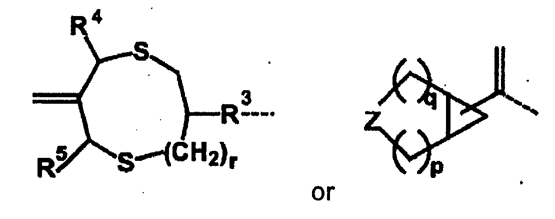

4. Dental material according to one of the preceding claims, in which at least one of the variables has one of the following meanings:
X¹ = is absent, O, S, an ester, amide or urethane group;
X² = is absent, O, S, an ester, amide or urethane group;
Y = is absent, O, S, an ester, amide or urethane group;
R = an (n+m)-valent organic group containing 1 to 20 carbon atoms and 0 to 8 hetero atoms, the H atoms of which optionally being totally or partially substituted by F atoms;
R¹ = is absent or a C₁-C₁₂ alkylene group that can be interrupted by O atoms;
R² = is absent or a C₁-C₁₂ alkylene group that can be interrupted by O atoms;
n = 1, 2 or 3;
m = 1,2 or 3;
HG = -P=O(OH)₂; -O-P=O(OH)₂ or -SO₂OH.

5. Dental material according to Claim 4, in which at least one of the variables has one of the following meanings:
X¹ = is absent or an ester group;
X² = is absent;
Y = is absent;
R = an aliphatic organic group containing 2 to 12 carbon atoms, the H atoms of which optionally being totally or partially substituted by F atoms, or phenylene;
R¹ = is absent;
R² = is absent, methylene or ethylene;
n = 1 or 2;
m = 1;
HG = -P=O(OH)₂; -O-P=O(OH)₂ or -SO₂OH.

6. Dental material according to one of the preceding claims, further comprising an initiator for radical polymerisation.

7. Dental material according to one of the preceding claims, further comprising an additional radically polymerisable monomer.

8. Dental material according to Claim 7, comprising at least one radically polymerisable monomer containing two or more radically polymerisable groups.

9. Dental material according to Claim 8, comprising at least one monomer that is selected from bi- or multifunctional acrylates and methacrylates, bisphenol-A di(meth)acrylate, bis-GMA (an addition product of methacrylic acid and bisphenol-A diglycidyl ether), UDMA (an addition product of 2-hydroxyethyl methacrylate and 2,2,4-trimethyl hexamethylene diisocyanate), di-, tri- or tetraethylene glycol di(meth)acrylate, trimethylolpropane tri(meth)acrylate, pentaerythritol tetra(meth)acrylate, butanediol di(meth)acrylate, 1,10-decanediol di(meth)acrylate or 1,12-dodecanediol di(meth)acrylate.

10. Dental material according to one of claims 7 to 8, comprising at least one radically ring-opening polymerisable monomer.

11. Dental material according to Claim 10, comprising at least one monomer that is selected from mono- and multifunctional vinyl cyclopropanes, bicyclic cyclopropane derivatives or cyclic allyl sulphides.

12. Dental material according to one of claims 7 to 11, comprising at least one additional radically polymerisable, acid group-containing monomer.

13. Dental material according to one of the preceding claims, additionally comprising a filler.

14. Dental material according to one of the preceding claims, further comprising at least one additive that is selected from stabilisers, UV-absorbers, dyes, pigments, solvents and lubricants.

15. Dental material according to one of the preceding claims, comprising:
(a) 0.5 to 30 wt.% acidic ring-opening polymerisable monomer according to Formula (I),
(b) 0.01 to 5 wt.% initiator,
(c) 5 to 90 wt.% additional radically polymerisable monomer,
(d) 0 to 85 wt.% filler,
(e) optionally 0.01 to 5 wt.% additive and
(f) 0 to 50 wt.% solvent.

16. Dental material according to Claim 15, comprising 5 to 90 wt.% additional ring-opening polymerisable monomer and 0 to 50 wt.% multifunctional methacrylate as the further monomer (c).

17. Use of a radically polymerisable monomer according to the general Formula (I) for manufacturing a dental material.

18. Use according to Claim 17, wherein the dental material is a composite, cement, adhesive or coating material.

19. Process for the preparation of a moulded article, in which a dental material according to one of claims 1 to 16 is moulded into an article with the desired shape, and then partially or completely cured.

## Revendications

1. Matériau dentaire qui contient au moins un monomère apte à la polymérisation radicalaire, correspondant à la formule générale (I) 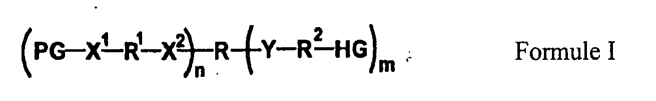 dans laquelle
PG est un groupe cyclique polymérisable avec ouverture de cycle, correspondant à l'une des formules suivantes : 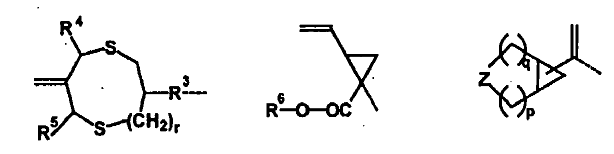 dans lesquelles
Z = O, S, 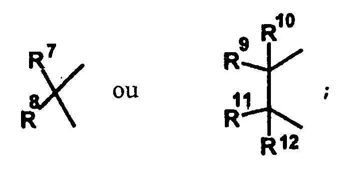
R³ est absent ou représente un radical alkylène en C₁-C₁₆ qui peut être interrompu par des atomes d'oxygène ;
R⁴ = H ou un radical alkyle en C₁-C₁₀;
R⁵ = H ou un radical alkyle en C₁-C₁₀;
R⁶ = H, un radical alkyle en C₁-C₁₀, le radical phényle ou benzyle ;
R⁷ = H, CN, CO-OR¹³ -CO-R¹³ ou R¹³;
R⁸ = H_{,} CN, CO-OR¹³ -CO-R¹³ ou R¹³ ;
R⁹-R¹² représentent chacun, indépendamment les uns des autres, H, -CO-OR¹³,-CONHR¹³, -CO-R¹³, un radical alkyle en C₁-C₁₅ qui peut être interrompu par O, un radical cycloaliphatique en C₄-C₁₂, un radical bicyclique en C₅-C₁₂, un radical aryle en C₆-C₁₄ ou un radical alkylaryle en C₇-C_{2O} ;
R¹³ = un radical alkyle en C₁-C₁₅ qui peut être interrompu par O, un radical cycloaliphatique en C₄-C₁₂, un radical bicyclique en C₅-C₁₂, un radical aryle en C₆-C₁₄ ou un radical alkylaryle en C₇-C₂₀, plusieurs radicaux R¹³ pouvant être identiques ou différents ;
q = 0, 1, 2 ou 3 ;
p = 0, 1, 2 ou 3
r = 0 ou 1 ;
X¹ est absent ou représente O, S, un groupe ester, amido ou uréthanne ;
X² est absent ou représente O, S, un groupe ester, amido ou uréthanne ;
Y est absent ou représente O, S, un groupe ester, amido ou uréthanne ;
R = un radical organique (n+m)-valent ayant de 1 à 35 atomes de carbone et 0 à 8 hétéroatomes, dont les atomes d'hydrogène peuvent être en partie ou en totalité remplacés par des atomes de fluor ;
R¹ est absent ou représente un radical alkylène en C₁-C₁₆ qui peut être interrompu par des atomes d'oxygène ;
R² est absent ou représente un radical alkylène en C₁-C₁₆ qui peut être interrompu par des atomes d'oxygène ;
n = 1, 2, 3, 4, 5 ou 6 ;
m = 1, 2 ou 3 ;
HG = -P=O(OH)₂ ; -P=O(OH) (OR¹⁴) ; -O-P=O(OH)₂ ; -O-P=O(OH)(OR¹⁵) ou -SO₂OH, où
R¹⁴ = un radical alkyle en C₁-C₁₅, le radical phényle ou benzyle ;
R¹⁵ = un radical alkyle en C₁-C₁₅, le radical phényle ou benzyle.

2. Matériau dentaire selon la revendication 1, dans lequel au moins l'une des variables a l'une des significations préférées suivantes :
R³ est absent ou représente un radical alkylène en C₁-C₁₀ qui peut être interrompu par des atomes d'oxygène ;
R⁴ = H ou un radical alkyle en C₁-C₅ ;
R⁵= H ou un radical alkyle en C₁-C₅ ;
R⁶ = H, un radical alkyle en C₁-C₅, le radical phényle ou benzyle ;
R⁷ = H, CO-OR¹³, -CO-R¹³ ou R¹³ ;
R⁸ = H, CO-OR¹³ -CO-R¹³ ou R¹³ ;
R⁹-R¹² représentent chacun, indépendamment les uns des autres, H, -CO-OR¹³, -CO-R¹³, un radical alkyle en C₁-C₁₅ qui peut être interrompu par O, un radical cycloaliphatique en C₄-C₆, un radical bicyclique en C₅-C₁₂, un radical phényle ou benzyle ;
R¹³ = un radical alkyle en C₁-C₁₀ qui peut être interrompu par O, un radical cycloaliphatique en C₄-C₆, un radical phényle ou benzyle, plusieurs radicaux R¹³ pouvant être identiques ou différents ;
q= 0, 1 ou 2 ;
p = 0, 1 ou 3 ;
r = 0 ou 1.

3. Matériau dentaire selon la revendication 1 ou 2, dans lequel PG est un groupe de formule

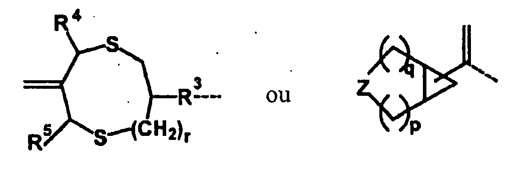

4. Matériau dentaire selon l'une quelconque des revendications précédentes, dans lequel au moins l'une des variables a l'une des significations préférées suivantes :
X¹ est absent ou représente O, un groupe ester, amido ou uréthanne ;
X² est absent ou représente O, un groupe ester, amido ou uréthanne ;
Y est absent ou représente O, un groupe ester, amido ou uréthanne ;
R = un radical organique (n+m)-valent ayant de 1 à 20 atomes de carbone et 0 à 8 hétéroatomes, dont les atomes d'hydrogène peuvent être en partie ou en totalité remplacés par des atomes de fluor ;
R¹ est absent ou représente un radical alkylène en C₁-C₁₂ qui peut être interrompu par des atomes d'oxygène ;
R² est absent ou représente un radical alkylène en C₁-C₁₂ qui peut être interrompu par des atomes d'oxygène ;
n = 1, 2 ou 3 ;
m = 1, 2 ou 3 ;
HG = -P=O(OH)₂ ; -O-P=O(OH)₂ ou -SO₂OH.

5. Matériau dentaire selon la revendication 4, dans lequel au moins l'une des variables a l'une des significations préférées suivantes :
X¹ est absent ou représente un groupe ester ;
X² est absent ;
Y est absent ;
R = un radical organique aliphatique ayant de 2 à 12 atomes de carbone, dont les atomes d'hydrogène peuvent être en partie ou en totalité remplacés par des atomes de fluor, ou le groupe phénylène ;
R¹ est absent ;
R² est absent ou représente un radical méthylène ou éthylène ;
n = 1 ou 2 ;
m = 1 ;
HG = -P=O(OH)₂ ; -O-P=O(OH)₂ ou-SO₂OH.

6. Matériau dentaire selon l'une quelconque des revendications précédentes, qui contient en outre un amorceur pour la polymérisation radicalaire.

7. Matériau dentaire selon l'une quelconque des revendications précédentes, qui en outre contient au moins un autre monomère apte à la polymérisation radicalaire.

8. Matériau dentaire selon la revendication 7, qui contient au moins un monomère apte à la polymérisation radicalaire, comportant deux ou plus de deux groupes aptes à la polymérisation radicalaire.

9. Matériau dentaire selon la revendication 8, qui contient au moins un monomère choisi parmi des acrylates et méthacrylates bi- ou polyfonctionnels, le di(méth)acrylate de bisphénol A, le bis-GMA (un produit d'addition d'acide méthacrylique et d'éther diglycidylique de bisphénol A), l'UDMA (un produit d'addition de méthacrylate de 2-hydroxyéthyle et de 2,2,4-triméthylhexaméthylènediisocyanate), le di(méth)acrylate de di-, tri- ou tétraéthylèneglycol, le tri(méth)acrylate de triméthylolpropane, le tétra(méth)acrylate de pentaérythritol, le di(méth)acrylate de butanediol, le di(méth)acrylate de 1,10-décanediol, le di(méth)acrylate de 1,12-dodécanediol.

10. Matériau dentaire selon la revendication 7 ou 8, qui contient au moins un monomère apte à la polymérisation radicalaire avec ouverture de cycle.

11. Matériau dentaire selon la revendication 10, qui contient au moins un monomère choisi parmi des vinylcyclopropanes mono- ou multifonctionnels, des dérivés bicycliques de cyclopropane et des sulfures d'allyle cycliques.

12. Matériau dentaire selon l'une quelconque des revendications 7 à 11, qui contient au moins un autre monomère apte à la polymérisation radicalaire et contenant des groupes acides,.

13. Matériau dentaire selon l'une quelconque des revendications précédentes, qui en outre contient au moins une charge.

14. Matériau dentaire selon l'une quelconque des revendications précédentes, qui en outre contient au moins un additif choisi parmi des stabilisants, des absorbeurs UV, des colorants, des pigments, des solvants et des lubrifiants.

15. Matériau dentaire selon l'une quelconque des revendications précédentes, qui contient
(a) 0,5 à 30 % en poids de monomère acide polymérisable avec ouverture de cycle, correspondant à la formule (I),
(b) 0,01 à 5 % en poids d'amorceur,
(c) 5 à 90 % en poids d'un autre monomère apte à la polymérisation radicalaire,
(d) 0 à 85 % en poids de charge,
(e) éventuellement 0,01 à 5 % en poids d'additif et
(f) 0 à 50 % en poids de solvant.

16. Matériau dentaire selon la revendication 15, qui contient comme autre monomère (c) 5-90 % en poids d'un monomère supplémentaire polymérisable avec ouverture de cycle et 0-50 % en poids d'un (méth)acrylate polyfonctionnel.

17. Utilisation d'un monomère apte à la polymérisation radicalaire, correspondant à la formule générale (I), pour la production d'un matériau dentaire.

18. Utilisation selon la revendication 17, dans laquelle le matériau dentaire est un composite, un ciment, un adhésif ou un matériau de revêtement.

19. Procédé pour la production d'un corps moulé, dans lequel un matériau dentaire selon l'une quelconque des revendications 1 à 16 est moulé à la forme désirée et ensuite partiellement ou totalement durci.
